# EUROPEAN PATENT SPECIFICATION

(11) **EP 1 854 499 B1**
(45) Date of publication and mention of the grant of the patent: **16.12.2009**
(21) Application number: 07251892.1
(22) Date of filing: 04.05.2007
(51) Int. Cl.: A61M 25/00, A61M 39/00

(54) **Catheter having reinforcing rings and method of use**
Katheter mit Verstärkungsringen und Verwendungsverfahren
Cathéter doté d'anneaux de renfort et procédé d'utilisation

(30) Priority: 08.05.2006 US 382165
(43) Date of publication of application: 14.11.2007
(73) Proprietor: Codman & Shurtleff, Inc., Raynham, Massachusetts 02767 (US)
(72) Inventor: Kraus, Robert G., Attleboro, MA 02703 (US)
(74) Representative: Tunstall, Christopher Stephen

(56) References cited:
- EP-A2- 0 670 169
- DE-C- 835 239
- US-A- 2 686 962
- US-A- 3 466 067
- US-A- 5 320 630

## Description

The present invention relates to a catheter having reinforcing rings. More particularly, the present invention relates to a catheter having reinforcing rings that can be cut to length after surgical placement and securely connected to a barbed connector.

There are many applications in which a catheter is fluidly connected to a connector. In medical applications, a catheter is often simply pushed over a barbed connector. But this connection may fail if, for example, an inadvertent force is applied to pull the catheter away from the connector. Thus, some have attempted to solve this problem by using an additional connector element radially about the catheter. However these additional elements increase the outer profile of the connection. Additionally, in many embodiments, the additional element is made of a hard plastic material that cannot be treated with currently available antimicrobial impregnation processes. These elements also prevent the surgeon from cutting the length of the catheter to size after it has been surgically placed at a desired location in the body. Thus, the surgeon must make an educated guess at the length of catheter needed before placing it within the body. If the surgeon guesses wrong at the length, removal of that catheter and reinsertion of another catheter is required.

In some instances, the surgeon will suture a catheter that has been connected to a barbed connector to maintain the connection over time. However, suturing a silicone rubber catheter to a barbed connector results in an inconsistent connection. If the suture is too tight, a cut to the catheter can cause leakage and tensile failure. If the suture is too loose, the catheter can be pulled loose and disconnect. Sutures are also subject to degradation over time and can fail years later after being placed within the body. Also, suturing a right angle connection requires the catheter to be lifted away from its preferred location within the body to provide a region for suturing. This is especially true for current shunt housings for treating hydrocephalus that use a right angle connection to the ventricular catheter. After suturing, the catheter is then replaced or pushed further into the body, such as, for example, brain tissue when a ventricular catheter is connected to a shunt housing.

US patent 3,466,067 discusses an elastic ring providing a radial compression force to couple a flexible tubular member and a rigid tubular member.

Thus, there is a need in the art for a catheter that has a low outer profile, which can be treated with an antimicrobial impregnation process, and that can be cut to length after surgical placement and securely connected to a barbed connector, without requiring moving the catheter once it has been placed at the desired location within the body.

### SUMMARY OF THE INVENTION

In accordance with a currently preferred exemplary embodiment, the present invention catheter includes a sleeve having a first end and a second end. Reinforcing rings are embedded in the sleeve at the first end.

In accordance with a currently preferred exemplary embodiment, the present invention connector assembly includes a sleeve having a first end and a second end. Reinforcing rings are embedded in the sleeve at the first end. A connector, which has a barbed end, is fluidly connected to the first end of the catheter.

### BRIEF DESCRIPTION OF THE DRAWINGS

The above and still further objects, features and advantages of the present invention will become apparent upon consideration of the following detailed description of a specific embodiment thereof, especially when taken in conjunction with the accompanying drawings wherein like reference numerals in the various figures are utilized to designate like components, and wherein:
Figure 1 is a perspective view of a catheter having reinforcing rings used with a shunt housing;
Figure 2 is a partial side view of a right angle connection;
Figure 3 is a cross-sectional view taken along line 3-3 of Fig. 2 and looking in the direction of the arrows; and
Figure 4 is a partial perspective view, with parts broken away, of a catheter having reinforcing rings.

### DETAILED DESCRIPTION OF THE CURRENTLY PREFERRED EXEMPLARY EMBODIMENT

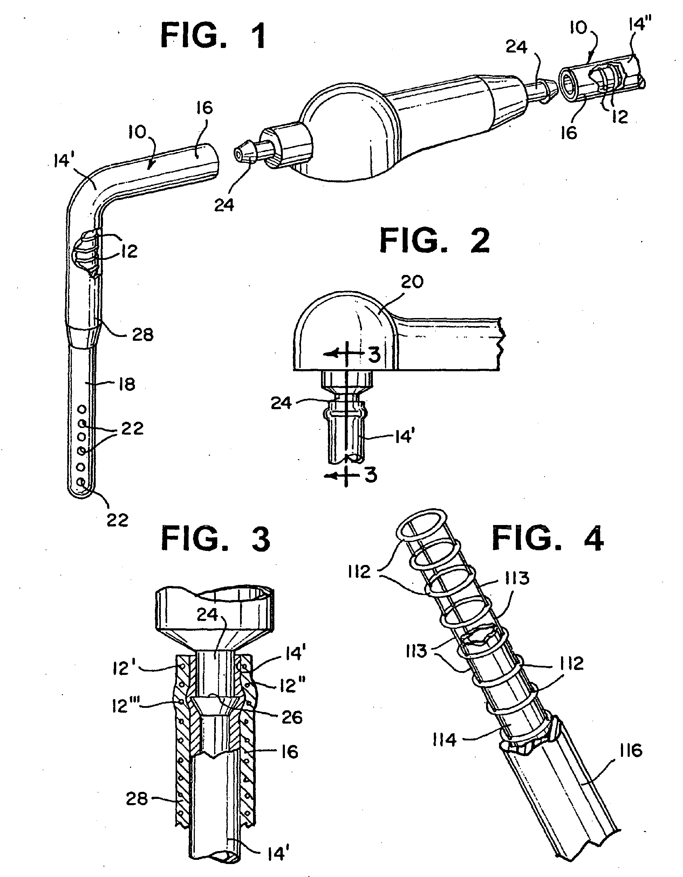

Referring now to Figs. 1-4, a device and method of using a catheter 10, which has reinforcing rings 12, in accordance with the present invention, is illustrated.

Catheter 10 is in the form of a sleeve 14 and has a first end 16 and a second end 18. A plurality of reinforcing rings 12 are embedded in sleeve 14 at its first end 16. In a currently preferred exemplary embodiment, the catheters are a ventricular catheter 14' and a drainage catheter 14" for use with a hydrocephalus shunt valve 20. Ventricular catheter 14' has at its second end 18 a plurality of inlet openings 22. Inlet openings 22 permit CSF to be drained from the patient's ventricles into the ventricular catheter and delivered to shunt valve 20. Shunt valve 20 regulates the amount of fluid to be drained from the ventricles and then to be delivered to the drainage catheter 14". Drainage catheter 14" delivers the cerebrospinal fluid ("CSF") to a portion of the body that can absorb the CSF. CSF is normally absorbed by the body's venous system. The lower end of the distal catheter of the shunt can be led into the abdomen (ventriculo-peritoneal shunt), wherein CSF passes into the bloodstream.

Reinforcing rings 12 are illustrated as being disposed at regular intervals. However, the rings could be disposed at irregular intervals, such as, for example, becoming closer together as the rings approach the end of the catheter. Another example for spacing the rings at irregular intervals would be to avoid kinking at specific bends in the catheter. In a currently preferred exemplary embodiment there are at least twenty (20) reinforcing rings disposed at regular intervals at the first end of the catheter 10. In a currently preferred embodiment, the ventricular catheter 14' has about thirty (30) reinforcing rings, and the drainage catheter 14" has about ten (10). In some embodiments, the reinforcing rings could be disposed throughout the entire length of the catheter 14. This would be especially practical in embodiments where the length of the catheter is relatively short, such as, for example, less than a two or three inches in length.

Referring now to Fig. 3, at least four (4) reinforcing rings 12 are preferred to effect an adequate connection of the catheter to a barbed connector 24. Preferably, at least two (2) reinforcing rings 12 should pass the barb apex 26 to ensure and optimum attachment. The most proximal ring 12', near the cut end, may be exposed and may have less adherence to the catheter. Thus, the need to ensure that at least two rings 12 (ie., 12' and 12") pass over the barb apex 26. This attachment can be visualized by the surgeon if the catheter sleeve 14 is made of clear silicone. Reinforcing rings 12 preferably have a smaller inside diameter than the largest barb diameter, which is the barb apex.

The catheter sleeve 14 is preferably made of silicone. At least in the area of the reinforcing rings, sleeve 14 is at least partially transparent so that the reinforcing rings 12 can be seen. The reinforcing rings can be made of titanium, stainless steel, or even a rigid plastic material. In fact, just about any material can be used so long as it is MRI compatible and biocompatible. As shown in Fig. 3, the plurality of reinforcing rings 12 are positioned axially discreet from one another. Thus, ventricular catheter 14' can be relatively easily cut to length after surgical placement and securely connected to a barbed connector. The surgeon's blade can simply cut between adjacent reinforcing rings 12, while still preferably leaving at least four rings to ensure an adequate connection to the barbed connector.

Reinforcing rings 12 are preferably embedded in the sleeve 14 at its first end. Rings 12 can, in one embodiment be embedded in a separate sleeve 28. Sleeve 28 can be placed over the proximal end 16 of each catheter 14 and bonded thereto. Alternatively, the reinforcing rings 12 can be placed on the outer surface of the proximal end 16 of catheter 14 with a slight interference fit. Silicone could then be applied to the outer surface of the proximal end of catheter 14 by, for example, using a dipping process or over molding process. The resulting structure would be a plurality of reinforcing rings embedded in the sleeve at its proximal end.

Referring now to Figure 4, an alternate embodiment of the reinforcing rings is illustrated. The reinforcing rings 112 can be made as one part, such as, for example, by injection molding. Each ring 112 is connected to an adjacent ring by three attachments 113. The three attachments 113 are frangible by a scalpel. Of course, the three attachments may limit flexibility of the catheter and would be harder to cut than just silicone.

A method of connecting a catheter to a barbed connector, where the catheter is comprised of a sleeve having a first end and a second end, and a plurality of reinforcing rings are embedded in the sleeve at the first end, is described below.

During surgery, the first end can be cut to exact length after ideal surgical placement. The cut end of the catheter is held, possibly with a conforming tool. The barb connector on the shunt housing and the cut end are brought together such that the barb advances into the cut end of the catheter and at least two rings snap over the barb, thereby forming a solid attachment and seal. As the first end of the sleeve is placed over the barbed end of the connector a fluid connection is created between the catheter and the connector. The placing step is sufficient to place at least two reinforcing rings 12', 12" passed the apex of the barb. After the placing step, the surgeon can visually verify that at least two reinforcing rings passed the apex of the barb because, at least in the area of the reinforcing rings, the catheter is transparent.

Preferably the method in accordance with the present invention connects a ventricular catheter to a first connector on a shunt housing and connects a drainage catheter to a second connector on the shunt housing. The ventricular catheter is comprised of a sleeve having a first end and a second end, and a plurality of reinforcing rings are embedded in the sleeve at the first end of the ventricular catheter. The drainage catheter is comprised of a sleeve having a first end and a second end, and a plurality of reinforcing rings are embedded in the sleeve at the first end of the drainage catheter. The first and second connector each having a barbed end. The first end of the sleeve of the ventricular catheter is placed over the barbed end of the first connector to fluidly connect the ventricular catheter to the shunt housing. The first end of the sleeve of the drainage catheter is placed over the barbed end of the second connector to fluidly connect the drainage catheter to the shunt housing. The ventricular catheter is placed within the ventricles of the brain. Before the placing of the ventricular catheter, the ventricular catheter is cut to exact size between two reinforcing rings. This cutting step occurs before the step of placing the first end of the sleeve of the ventricular catheter over the barbed end of the first connector.

The catheter with the reinforcing rings embedded therein can be treated with an antimicrobial agent, such as is done with the Bactiseal® catheter that is currently sold by Codman & Shurtleff of Raynham, Mass.

Having described the presently preferred exemplary embodiment of an apparatus and a method of using a catheter having reinforcing rings that can be cut to length after surgical placement and securely connected to a barbed connector, it is believed that other modifications, variations and changes will be suggested to those skilled in the art in view of the teachings set forth herein. Substitutions of elements from one described embodiment to another are also fully intended and contemplated. It is also to be understood that the drawings are not necessarily drawn to scale, but that they are merely conceptual in nature. It is, therefore, to be understood that all such modifications, variations, and changes are believed to fall within the scope of the present invention as defined by the appended claims.

## Claims

1. A catheter (10) comprising:
a sleeve (14) having a first end (16) and a second end (18); and
a plurality of reinforcing rings (12);
**characterized in that** said plurality of reinforcing rings are embedded in the sleeve at said first end.

2. The catheter according to claim 1, wherein the plurality of reinforcing rings are disposed at regular intervals.

3. The catheter according to claim 1, wherein there are at least four reinforcing rings.

4. The catheter according to claim 1, wherein there are at least twenty reinforcing rings.

5. The catheter according to claim 1, wherein the sleeve is made of a silicone.

6. The catheter according to claim 5, wherein the silicone, at least in the area of the reinforcing rings, is at least partially transparent.

7. The catheter according to claim 1, wherein the plurality of reinforcing rings are made of titanium.

8. The catheter according to claim 1, wherein the plurality of reinforcing rings are made of stainless steel.

9. The catheter according to claim 1, wherein the plurality of reinforcing rings are made of a rigid plastic material.

10. The catheter according to claim 1, wherein the plurality of reinforcing rings are discreet from one another.

11. The catheter according to claim 2, wherein the plurality of reinforcing rings are discreet from one another.

12. The catheter according to claim 1, wherein the plurality of reinforcing rings are disposed at irregular intervals.

13. The catheter according to claim 1, wherein the plurality of reinforcing rings are interconnected as a one-piece assembly.

14. A connector assembly comprising:
the catheter of any one of claims 1-13 or 26; and
a connector (24) having a barbed end, said first end of said sleeve being connected to said barbed end.

15. A method of connecting a catheter (14) to a connector (24), wherein said catheter is comprised of a sleeve having a first end (16) and a second end (18), and a plurality of reinforcing rings (12);
**characterized in that** said plurality of reinforcing rings are embedded in the sleeve at the first end, the connector having a barbed end, said method comprising the step of:
placing the first end of the sleeve over the barbed end of the connector to fluidly connect the catheter to the connector.

16. The method according to claim 15, wherein the placing step is sufficient to place at least two reinforcing rings past the apex of the barb.

17. The method according to claim 16, wherein said method further comprising the step of, after the placing step, verifying that at least two reinforcing rings passed the apex of the barb.

18. The method according to claim 17, wherein the sleeve, at least in the area of the reinforcing rings, is at least partially transparent, said method further comprising the step of, after the placing step, visually verifying that at least two reinforcing rings passed the apex of the barb.

19. The method of claim 15, wherein the method comprises connecting a ventricular catheter (14') to a first connector on a shunt housing (20) and connecting a drainage catheter (14") to a second connector on the shunt housing, wherein said ventricular catheter is comprised of a sleeve having a first end and a second end, and a plurality of reinforcing rings are embedded in the sleeve at the first end of the ventricular catheter, said drainage catheter is comprised of a sleeve having a first end and a second end, and a plurality of reinforcing rings are embedded in the sleeve at the first end of the drainage catheter, the first and second connector each having a barbed end, said method comprising the step of:
placing the first end of the sleeve of the ventricular catheter over the barbed end of the first connector to fluidly connect the ventricular catheter to the shunt housing;
placing the first end of the sleeve of the drainage catheter over the barbed end of the second connector to fluidly connect the drainage catheter to the shunt housing.

20. The method according to claim 19, wherein both of the placing steps are sufficient to place at least two reinforcing rings past the apex of the barb.

21. The method according to claim 20, wherein said method further comprising the step of, after the placing steps, verifying that at least two reinforcing rings passed the apex of the barb.

22. The method according to claim 21, wherein the sleeve of both the ventricular catheter and the drainage catheter, at least in the area of the reinforcing rings, is at least partially transparent, said method further comprising the step of, after the pushing steps, visually verifying that at least two reinforcing rings past the apex of the barb.

23. The method according to claim 19, further comprising the step of cutting the ventricular catheter between two reinforcing rings.

24. The method according to claim 23, wherein the cutting step occurs before the step of placing the first end of the sleeve of the ventricular catheter over the barbed end of the first connector.

25. The catheter according to claim 1, wherein the catheter has an antimicrobial agent embedded therein.

## Patentansprüche

1. Katheter (10), der aufweist:
eine Hülse (14) mit einem ersten Ende (16) und einem zweiten Ende (18); und eine Vielzahl von Verstärkungsringen (12);
**dadurch gekennzeichnet, dass** die Vielzahl der Verstärkungsringe in die Hülse an dem ersten Ende eingebettet sind.

2. Katheter nach Anspruch 1, bei dem die Vielzahl der Verstärkungsringe in regelmäßigen Abständen angeordnet ist.

3. Katheter nach Anspruch 1, bei dem es wenigstens vier Verstärkungsringe gibt.

4. Katheter nach Anspruch 1, bei dem es wenigstens zwanzig Verstärkungsringe gibt.

5. Katheter nach Anspruch 1, bei dem die Hülse aus einem Silikon hergestellt ist.

6. Katheter nach Anspruch 5, bei dem das Silikon, wenigstens in dem Gebiet der Verstärkungsringe, zumindest teilweise transparent ist.

7. Katheter nach Anspruch 1, bei dem die Vielzahl der Verstärkungsringe aus Titan hergestellt ist.

8. Katheter nach Anspruch 1, bei dem die Vielzahl der Verstärkungsringe aus rostfreiem Stahl hergestellt ist.

9. Katheter nach Anspruch 1, bei dem die Vielzahl der Verstärkungsringe aus einem starren Kunststoffmaterial hergestellt ist.

10. Katheter nach Anspruch 1, bei dem die Vielzahl der Verstärkungsringe getrennt voneinander sind.

11. Katheter nach Anspruch 2, bei dem die Vielzahl der Verstärkungsringe getrennt voneinander sind.

12. Katheter nach Anspruch 1, bei dem die Vielzahl der Verstärkungsringe in unregelmäßigen Abständen angeordnet ist.

13. Katheter nach Anspruch 1, bei dem die Vielzahl der Verstärkungsringe zu einer einstückigen Anordnung verbunden ist.

14. Verbinderanordnung, die aufweist:
den Katheter nach einem der Ansprüche 1 - 13 oder 26; und
einen Verbinder (24) mit einem widerhakenähnlichen Ende, wobei das erste Ende der Hülse mit dem widerhakenähnlichen Ende verbunden ist.

15. Verfahren zum Verbinden eines Katheters (14) mit einem Verbinder (24), wobei der Katheter aus einer Hülse mit einem ersten Ende (16) und einem zweiten Ende (18) und einer Vielzahl von Verstärkungsringen (12) besteht;
**dadurch gekennzeichnet, dass** die Vielzahl der Verstärkungsringe in die Hülse an dem ersten Ende eingebettet ist, wobei der Verbinder ein widerhakenähnliches Ende hat, wobei das Verfahren den Schritt aufweist:
Aufschieben des ersten Endes der Hülse über das widerhakenähnliche Ende des Verbinders, um den Katheter fluidisch mit dem Verbinder verbinden.

16. Verfahren nach Anspruch 15, bei dem der Aufschiebeschritt ausreichend ist, um wenigstens zwei Verstärkungsringe hinter den Scheitel des Widerhakens zu bringen.

17. Verfahren nach Anspruch 16, wobei das Verfahren weiter den Schritt, nach dem Aufschiebeschritt, des Verifizierens, dass wenigstens zwei Verstärkungsringe über den Scheitel des Widerhakens gelaufen sind, aufweist.

18. Verfahren nach Anspruch 17, bei dem die Hülse, wenigstens in dem Bereich der Verstärkungsringe, zumindest teilweise transparent ist, wobei das Verfahren weiter den Schritt, nach dem Aufschiebeschritt, des visuellen Verifizierens, das wenigstens zwei Verstärkungsringe über den Scheitel des Widerhakens gelaufen sind, aufweist.

19. Verfahren nach Anspruch 15, wobei das Verfahren das Verbinden eines ventrikularen Katheters (14') mit einem ersten Verbinder auf einem Shunt-Gehäuse (20) und das Verbinden eines Drainage-Katheters (14") mit einem zweiten Verbinder auf dem Shunt-Gehäuse aufweist, wobei der ventrikulare Katheter aus einer Hülse mit einem ersten Ende und einem zweiten Ende besteht und eine Vielzahl von Verstärkungsringen in die Hülse an dem ersten Ende des ventrikularen Katheters eingebettet ist, wobei der Drainage-Katheter aus einer Hülse mit einem ersten und einem zweiten Ende besteht und eine Vielzahl von Verstärkungsringen in die Hülse an dem ersten Ende des Drainage-Katheters eingebettet ist, wobei der erste und der zweite Verbinder jeder ein widerhakenähnliches Ende hat, wobei das Verfahren den Schritt aufweist:
Schieben des ersten Endes der Hülse des ventrikularen Katheters über das widerhakenähnliche Ende des ersten Verbinders, um den ventrikularen Katheter fluidisch mit dem Shunt-Gehäuse zu verbinden;
Schieben des ersten Endes der Hülse des Drainage-Katheters über das widerhakenähnliche Ende des zweiten Verbinders, um den Drainage-Katheter fluidisch mit dem Shunt-Gehäuse zu verbinden.

20. Verfahren nach Anspruch 19, bei dem beide Aufschiebeschritte ausreichend sind, um wenigstens zwei Verstärkungsringe hinter den Scheitel des Widerhakens zu bringen.

21. Verfahren nach Anspruch 20, wobei das Verfahren weiter den Schritt, nach den Aufschiebeschritten, des Verifizierens, dass wenigstens zwei Verstärkungsringe über den Scheitel des Widerhakens gelaufen sind, aufweist.

22. Verfahren nach Anspruch 21, bei dem die Hülse sowohl des ventrikularen Katheters als auch des Drainage-Katheters, wenigstens in dem Bereich der Verstärkungsringe, zumindest teilweise transparent ist, wobei das Verfahren weiter den Schritt, nach den Aufschiebeschritten, des visuellen Verifizierens, dass wenigstens zwei Verstärkungsringe über den Scheitel des Widerhakens gelaufen sind, aufweist.

23. Verfahren nach Anspruch 19, das weiter den Schritt des Schneidens des ventrikularen Katheters zwischen zwei Verstärkungsringen aufweist.

24. Verfahren nach Anspruch 23, bei dem der Schneidschritt vor dem Schritt des Aufschiebens des ersten Endes der Hülse des ventrikularen Katheters über das widerhakenähnliche Ende des ersten Verbinders geschieht.

25. Katheter nach Anspruch 1, wobei der Katheter ein in ihm eingebettetes antimikrobielles Mittel aufweist.

## Revendications

1. Cathéter (10) comprenant :
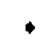 un manchon (14) ayant une première extrémité (16) et une seconde extrémité (18) ; et
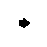 une pluralité d'anneaux de renfort (12) ;
**caractérisé en ce que** ladite pluralité d'anneaux de renfort est intégrée dans le manchon à ladite première extrémité.

2. Cathéter selon la revendication 1, dans lequel la pluralité d'anneaux de renfort est disposée à intervalles réguliers.

3. Cathéter selon la revendication 1, dans lequel il existe au moins quatre anneaux de renfort.

4. Cathéter selon la revendication 1, dans lequel il existe au moins vingt anneaux de renfort.

5. Cathéter selon la revendication 1, dans lequel le manchon est constitué de silicone.

6. Cathéter selon la revendication 5, dans lequel le silicone, au moins dans la zone des anneaux de renfort, est au moins partiellement transparent.

7. Cathéter selon la revendication 1, dans lequel la pluralité d'anneaux de renfort est constituée de titane.

8. Cathéter selon la revendication 1, dans lequel la pluralité d'anneaux de renfort est constituée d'acier inoxydable.

9. Cathéter selon la revendication 1, dans lequel la pluralité d'anneaux de renfort est constituée de matière plastique rigide.

10. Cathéter selon la revendication 1, dans lequel les anneaux de renfort sont distincts les uns des autres.

11. Cathéter selon la revendication 2, dans lequel les anneaux de renfort sont distincts les uns des autres.

12. Cathéter selon la revendication 1, dans lequel les anneaux de renfort sont disposés à intervalles irréguliers.

13. Cathéter selon la revendication 1, dans lequel les anneaux de renfort sont interconnectés comme un ensemble d'un seul tenant.

14. Ensemble connecteur comprenant :
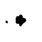 le cathéter selon l'une quelconque des revendications 1-13 ou 25 ; et
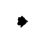 un connecteur (24) ayant une extrémité crénelée, ladite première extrémité dudit manchon étant connectée à ladite extrémité crénelée.

15. Méthode de connexion d'un cathéter (14) à un connecteur (24), dans laquelle ledit cathéter comprend un manchon ayant une première extrémité (16) et une seconde extrémité (18), et une pluralité d'anneaux de renfort (12) ;
**caractérisé en ce que** ladite pluralité d'anneaux de renfort est intégrée dans le manchon à la première extrémité, le connecteur ayant une extrémité crénelée, ladite méthode comprenant l'étape consistant à :
placer la première extrémité du manchon sur l'extrémité crénelée du connecteur pour connecter de manière fluide le cathéter au connecteur.

16. Méthode selon la revendication 15, dans laquelle l'étape de mise en place est suffisante pour placer au moins deux anneaux de renfort après le sommet de l'aiguillon.

17. Méthode selon la revendication 16, dans laquelle ladite méthode comprend en outre l'étape consistant, après l'étape de mise en place, à vérifier qu'au moins deux anneaux de renfort ont passé le sommet de l'aiguillon.

18. Méthode selon la revendication 17, dans laquelle le manchon, au moins dans la zone des anneaux de renfort, est au moins partiellement transparent, ladite méthode comprenant en outre l'étape consistant, après l'étape de mise en place, à vérifier visuellement qu'au moins deux anneaux de renfort ont passé le sommet de l'aiguillon.

19. Méthode selon la revendication 15, dans laquelle ladite méthode comprend la connexion d'un cathéter ventriculaire (14') à un premier connecteur sur un logement de pontage (20) et la connexion d'un cathéter de drainage (14'') à un second connecteur sur le logement de pontage, dans laquelle ledit cathéter ventriculaire comprend un manchon ayant une première extrémité et une seconde extrémité, et une pluralité d'anneaux de renfort sont intégrés dans le manchon à la première extrémité du cathéter ventriculaire, ledit cathéter de drainage comprend un manchon ayant une première extrémité et une seconde extrémité, et une pluralité d'anneaux de renfort sont intégrés dans le manchon à la première extrémité du cathéter de drainage, le premier et le second connecteurs ayant chacun une extrémité crénelée, ladite méthode comprenant l'étape consistant à :
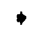 placer la première extrémité du manchon du cathéter ventriculaire sur l'extrémité crénelée du premier connecteur, de façon à relier de manière fluide le cathéter ventriculaire au logement de pontage ;
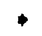 placer la première extrémité du manchon du cathéter de drainage sur l'extrémité crénelée du second connecteur afin de connecter de manière fluide le cathéter de drainage au logement de pontage.

20. Méthode selon la revendication 19, dans laquelle les deux étapes de mise en place sont suffisantes pour placer au moins deux anneaux de renfort après le sommet de l'aiguillon.

21. Méthode selon la revendication 20, dans laquelle ladite méthode comprend en outre l'étape consistant, après les étapes de mise en place, à vérifier qu'au moins deux anneaux de renfort ont passé le sommet de l'aiguillon.

22. Méthode selon la revendication 21, dans laquelle le manchon du cathéter ventriculaire et du cathéter de drainage, au moins dans la zone des anneaux de renfort, est au moins partiellement transparent, ladite méthode comprenant en outre l'étape, après les étapes de poussée, consistant à vérifier visuellement qu'au moins deux anneaux de renfort ont passé le sommet de l'aiguillon.

23. Méthode selon la revendication 19, comprenant en outre l'étape consistant à couper le cathéter ventriculaire entre deux anneaux de renfort.

24. Méthode selon la revendication 23, dans laquelle l'étape de découpe survient avant l'étape de mise en place de la première extrémité du manchon du cathéter ventriculaire sur l'extrémité crénelée du premier connecteur.

25. Cathéter selon la revendication 1, dans lequel le cathéter a un agent antimicrobien intégré à l'intérieur.
